# EUROPÄISCHE PATENTSCHRIFT

(11) **EP 1 436 609 B1**
(45) Veröffentlichungstag und Bekanntmachung des Hinweises auf die Patenterteilung: **18.03.2009**
(21) Anmeldenummer: 02781236.1
(22) Anmeldetag: 10.10.2002
(51) Int. Cl.: G01N 30/00, G01N 33/53, C12N 15/10, B01J 19/00, C12Q 1/68

(54) **MIKROFLUIDISCHES EXTRAKTIONSVERFAHREN**
MICROFLUIDIC EXTRACTION METHOD
PROCEDE D'EXTRACTION MICROFLUIDIQUE

(30) Priorität: 10.10.2001 DE 10149947
(43) Veröffentlichungstag der Anmeldung: 14.07.2004
(73) Patentinhaber: febit holding GmbH, 69120 Heidelberg (DE)
(72) Erfinder: STÄHLER, Cord, F., 69469 Weinheim (DE); STÄHLER, Peer, F., 68167 Mannheim (DE); MÜLLER, Manfred, 69198 Schriesheim (DE)
(74) Vertreter: Weiss, Wolfgang
(86) Internationale Anmeldenummer: PCT/EP2002/011383
(87) Internationale Veröffentlichungsnummer: WO 2003/031965

(56) Entgegenhaltungen:
- EP-A- 0 846 776
- WO-A-01/40509
- WO-A-02/12855
- WO-A-02/052045
- ERICSON C ET AL: "ELECTROOSMOSIS- AND PRESSURE-DRIVEN CHROMATOGRAPHY IN CHIPS USING CONTINUOUS BEDS" ANALYTICAL CHEMISTRY.A, AMERICAN CHEMICAL SOCIETY. COLUMBUS, US, Bd. 72, Nr. 1, 1. Januar 2000 (2000-01-01), Seiten 81-87, XP002163649
- BING HE AND FRED REGNIER: "Microfabricated liquid chromatography columns base on collocated monolith support structures" JOURNAL OF PHARMACEUTICAL AND BIOMEDICAL ANALYSIS, Bd. 17, 1. September 1998 (1998-09-01), Seiten 925-932, XP002255566
- JÖRG P. KUTTER ET AL.: "Solvent-Programmed Microchip Open-Channel Electrochromatography" ANALYTICAL CHEMISTRY, Bd. 70, 1. August 1998 (1998-08-01), Seiten 3291-3297, XP002255567
- BRUIN G J M: "RECENT DEVELOPMENTS IN ELECTROKINETICALLY DRIVEN ANALYSIS ON MICROFABRICATED DEVICES" ELECTROPHORESIS, WEINHEIM, DE, Bd. 21, 2000, Seiten 3931-3951, XP001031800 ISSN: 0173-0835
- HUI GE: "UPA, a universal protein array system for quantitative detection of protein-protein, protein-DNA, protein-RNA and protein-ligand interactions" NUCLEIC ACID RESEARCH, Bd. 28, Nr. 2, 2000, Seiten E3(I)-E3(VII),
- OKANO K.; YASUDA K.; ISHIWATA S.: 'Position-specific release of DNA from a chip by using photothermal denaturation' SENSORS AND ACTUATORS B Bd. 64, Nr. 1-3, 01 Juni 2000, ELSEVIER SEQUOIA S.A., LAUSANNE, CH, Seiten 88 - 94, XP004199291
- LOLLO B.A. ET AL: 'IMPROVED TWO-DIMENSIONAL GEL ELECTROPHORESIS REPRESENTATION OF SERUM PROTEINS BY USING PROTOCLEAR TM' ELECTROPHORESIS Bd. 20, Nr. 4/05, 01 April 1999, DE, Seiten 854 - 859, XP001006007
- RAYMACKERS J. ET AL: 'IDENTIFICATION OF TWO-DIMENSIONALLY SEPARATED HUMAN CEREBROSPINAL FLUID PROTEINS BY N-TERMINAL SEQUENCING, MATRIX-ASSISTED LASER DESORPTION/IONIZATION-MASS SPECTROMETRY, NANOLIQUID CHROMATOGRAPHY-ELECTROSPRAY IONIZATION-TIME OF FLIGHT-MASSSPECTROMETRY, AND TANDEM MASS SPECTROMETRY' ELECTROPHORESIS Bd. 21, Nr. 11, 01 Juni 2000, DE, Seiten 2266 - 2283, XP008001622

## Beschreibung

Die Erfindung betrifft ein Extraktionsverfahren zur Isolierung von Zielmolekülen aus einer Probe unter Verwendung eines mikrofluidischen Trägers.

Die selektive Extraktion von Molekülen ist für viele Arbeitsgebiete in der Biochemie, Biologie und Medizin ein zentraler und wichtiger Prozess. Zu diesen Arbeitsgebieten gehört die Extraktion und Aufreinigung von Nukleinsäuren, Proteinen, Zuckern und weiteren biochemischen funktionellen Molekülen.

Auch in der Genetik spielen biochemische Methoden zur Extraktion von bestimmten Molekülen, Verbindungen oder Stoffklassen eine wichtige Rolle. Besondere Bedeutung kommt dabei der Aufreinigung von Nukleinsäuren, meist DNA und RNA, zu. Wesentliche Schritte der Rekombinationstechnik setzen die Isolation und Reinigung von bestimmten Nukleinsäuren, z.B. von Plasmid-DNA, voraus. Auch die Konstruktion von Gen-Bibliotheken aus messenger-RNA (mRNA), die eine zentrale Bedeutung in der Gentechnik hat, ist auf die Isolation einer gewünschten RNA-Population angewiesen. Aus solchen Bibliotheken werden Gene oder Genfragmente "gefischt", um sie weiter untersuchen oder manipulieren zu können.

Biochemische, biologische und medizinische Analyseverfahren lassen sich durch Miniaturisierung und Parallelisierung in ihrer Effizienz und Aussagekraft enorm steigern. Solche Miniaturisierungen betreffen z.B. die Analyse von genetischem Material mit Hilfe von Hybridisierungsexperimenten auf DNA-Mikroarrays. Durch Entwicklung von Mikroarrays aus geeigneten DNA-Sonden als Rezeptoren lassen sich ganze Genome und Transkriptome analysieren. Für die Herstellung und Anwendung der Mikroarrays spielen Extraktionsverfahren eine wichtige Rolle und sind zugleich ein wichtiger Kostenfaktor, wenn es sich um solche Mikroarrays handelt, bei der die entsprechenden DNA-Polymersonden unabhängig vom Reaktionsträger synthetisiert ("off chip" Synthese) und dann auf den Reaktionsträger aufgebracht werden. Neben den recht weit verbreiteten DNA-Mikroarrays sind auch Verfahren miniaturisiert und parallelisiert worden, die dem Screening nach Molekülen mit besonderen Eigenschaften dienen; z.B. Ribozymen. Weitere Beispiele für Rezeptoren auf Mikroarrays sind Proteine und solche Moleküle, die in der Natur nicht vorkommen, wie z.B. Peptidnukleinsäuren (PNA). Viele solcher Assay-Formate werden unter der Rubrik Biochips zusammengefasst. Für nahezu alle diese Assay-Formate und Biochips sind die Isolation und Reinigung des Probenmaterials zentrale Prozesse der Probenvorbereitung.

Mikroreaktionstechniken können mit solchen Mikroarrays gekoppelt werden, um sowohl bei Probenvorbereitung als auch bei Herstellung des eigentlichen Arrays zu schnellen und effizienten Systemen zu kommen. Dies schließt auch die Verwendung von mikrofluidischen Verfahren mit ein.

Für die biochemische Isolation von Nukleinsäuren stehen zahlreiche Methoden zur Verfügung. Die Methoden erlauben zwar die Isolation und gegebenenfalls auch Aufreinigung von RNA oder DNA über ihre biophysikalischen Eigenschaften, sind dabei aber völlig unspezifisch in Bezug auf die Sequenz des Nukleinsäurestranges.

So können aus Gesamt-RNA, die unterschiedliche Sorten RNA enthält, mRNA-Moleküle relativ spezifisch isoliert werden, indem man auf einer festen Phase, z.B. Latex-Beads, Magnet-Beads, Controlled Pore Glass-Beads oder der Matrix einer Säule Poly-Thymidin Stränge (poly-T Stränge) immobilisiert. Diese Poly-T Stränge hybridisieren nach Zugabe von Gesamt-RNA mit dem Poly-Adenin (poly-A) Schwanz von mRNA-Molekülen und erlauben das Abtrennen der nichtgebundenen RNA-Moleküle. Die mRNA-Moleküle werden dann isoliert, indem der Puffer entsprechend so geändert wird, dass die Hybridisierung zugunsten von Einzelsträngen aufgehoben wird. Die frei werdenden mRNA-Moleküle kann man anschließend eluieren.

Der Informationsgehalt einer solchen Isolationsmatrix ist vergleichsweise gering, da nur zwei Kategorien von Zielmolekülen unterschieden werden können, nämlich mit oder ohne poly-A Schwanz.

Im Falle von Isolationsverfahren für Proteinen verhält es sich zumeist ähnlich. So werden Immunglobuline häufig durch eine Isolationsmatrix in einer Säule mit immobilisiertem Protein A (aus Staphylococcus aureus) isoliert (Brown et al., Biochem. Soc. Transactions (England) 26 (1998), 249). In anderen Versionen des Verfahrens werden Antikörper für eines oder wenige Zielmoleküle an die Isolationsmatrix gebunden.

Solche Isolationsverfahren werden allgemein als Affinitätschromatographie bezeichnet. Ein Nachteil dieser Verfahren besteht darin, dass keine parallele selektive Isolation unterschiedlicher Zielmoleküle möglich ist.

US-Patent 6,013,440 beschreibt ein Verfahren zur Herstellung einer Affinitätsmatrix, wobei ein Satz unterschiedlicher Nukleinsäuresonden auf einem festen Träger immobilisiert wird, um auf diese Weise Zielnukleinsäuren einer noch unbekannten Sequenz aus einer Probe anzureichern. Neben anderen Nachteilen ist hervorzuheben, dass das vorgeschlagene Verfahren flächige Träger als Isolationsmatrix verwendet, die nur eine ungünstige Elution erlauben. Auch mit diesem Verfahren können somit die aus dem Stand der Technik bekannten Nachteile nicht beseitigt werden.

In der vorliegenden Offenbarung werden ein Verfahren und eine Vorrichtung zur Verfügung gestellt, bei denen ein mikrofluidischer Träger als Isolationsmatrix verwendet wird, der eine selektive Isolation von bestimmten biochemischen funktionellen Molekülen (Zielmolekülen), insbesondere eine selektive parallele Isolation mehrerer Spezies von Zielmolekülen, aus einem Gemisch erlaubt. Das Verfahren beinhaltet eine selektive Isolation von biochemisch funktionellen Zielmolekülen durch Bindung an ein Substrat mit immobilisierten Rezeptoren als Interaktionspartner. Die Funktionalität eines Zielmoleküls ist durch seine Fähigkeit zur selektiven Bindung an einen für das Zielmolekül spezifischen Rezeptor, vorzugsweise durch bioaffine Wechselwirkungen, wie Hybridisierung, Rezeptor-Lingand-Bindung, Antigen-Antikörper-Bindung, Saccharid-Lectin-Bindung etc, definiert.

Das erfindungsgemäße Verfahren ist in den Ansprüchen definiert. Es bedient sich eines mikrofluidischen Trägers mit einem Array selektiv bindender Rezeptoren bzw. Fängersonden als Isolationsmatrix für die Zielmoleküle. In einer bevorzugten Ausführungsform erfolgt eine in situ Synthese der Rezeptoren auf oder in dem mikrofluidischen Reaktionsträger. Diese Vorgehensweise bei der Herstellung der Isolationsmatrix erlaubt bei Einsatz entsprechender Verfahren für die in situ Synthese einen sehr hohen Informationsgehalt der Isolationsmatrix. Im Fall von Nukleinsäuren als Zielmoleküle kann ein geeignetes System für die in situ Synthese der entsprechenden Fängersonden Tausende von definierten Sequenzen an der Isolationsmatrix herstellen. Somit wird durch die Erfindung ein Weg eröffnet, um Hunderte bis Tausende von individuellen DNA- oder RNA-Molekülen gezielt aus einem Gemisch zu extrahieren.

Der die immobilisierten Rezeptoren enthaltende Träger kann z.B. zum Einsatz kommen in der akademischen Forschung, der Grundlagenforschung, der industriellen Forschung, in der Qualitätskontrolle, in der Pharmaforschung, in der Biotechnologie, in der klinischen Forschung, in der klinischen Diagnostik, in Screening-Verfahren, in der patientenindividuellen Diagnostik, in klinischen Studien, in der Forensik, für genetische Tests, wie Elternschaftsbestimmungen, in der Tier- und Pflanzenzucht oder im Umweltmonitoring.

Gegenstand der Erfindung ist somit ein Verfahren zur Isolierung von Zielmolekülen aus einer Probe gemäß Anspruch 1.

Die durch das erfindungsgemäße Verfahren isolierten Zielmoleküle werden vorzugsweise aus biologischen Polymeren, wie Nukleinsäuren, z.B. doppelsträngigen oder einzelsträngigen DNA-Molekülen, z.B. genomischen DNA-Molekülen oder cDNA-Molekülen, oder RNA-Molekülen, Polypeptiden, wie etwa Proteinen, Glykoproteinen, Lipoproteinen, Nukleoproteinen etc., Peptiden und Sacchariden ausgewählt.

Bevorzugt handelt es sich bei den Zielmolekülen um Nukleinsäuren und die Rezeptoren auf dem mikrofluidischen Träger werden aus dazu komplementären Hybridisierungssonden ausgewählt. Bei den Hybridisierungssonden kann es sich ebenfalls um Nukleinsäuren, insbesondere DNA-Moleküle, jedoch auch um Nukleinsäureanaloga, wie Peptidnukleinsäuren (PNA), Locked-Nukleinsäuren (LNA) etc. handeln. Die Hybridisierungssonden haben vorzugsweise eine Länge entsprechend 10-100 Nukleotiden und müssen nicht durchgängig aus Bausteinen mit Basen bestehen, d.h. sie können beispielsweise auch abasische Bausteine, Linker, Spacer etc. enthalten. Die Hybridisierungssonden können am 3'-Ende, 5'-Ende oder dazwischen oder an mehreren Positionen am Träger gebunden sein.

Die Rezeptoren können auf dem mikrofluidischen Träger durch nichtkovalente oder kovalente Wechselwirkungen immobilisiert werden. Vorzugsweise erfolgt eine kovalente Immobilisierung, besonders bevorzugt über bi- oder polyfunktionelle Spacermoleküle. Alternativ ist eine Immobilisierung über elektrostatische Wechselwirkungen oder bioaffine Wechselwirkungen, z.B. Streptavidin/Biotin, Avidin/Biotin etc. bevorzugt.

Ein anderes bevorzugtes Beispiel für Rezeptoren sind Peptide. Desweiteren können selbstverständlich auch niedermolekulare Substanzbibliotheken als Rezeptoren eingesetzt werden.

Vorzugsweise werden die auf dem Träger immobilisierten Rezeptoren, z.B. die Hybridisierungssonden, durch in situ Synthese, z.B. durch schrittweisen Aufbau aus Synthesebausteinen, auf dem Träger erzeugt und sind damit frei wählbar. Die Bausteine für diese in situ Synthese können Monomere der betreffenden Stoffklasse sein, also im Fall von Nukleinsäuren z.B. Nukleotidbausteine. Es kann sich aber auch um komplexere Bausteine handeln, so z.B. Oligonukleotide oder Oligopeptide aus mehreren, z.B. 2, 3, oder 4, Monomereinheiten.

Die für das erfindungsgemäße Verfahren verwendete Probe ist vorzugsweise eine komplexe Probe, d.h. die Zielmoleküle müssen aus einer Vielzahl ähnlicher Molekülspezies selektiv isoliert werden. Die Probe kann eine biologische Probe sein, z.B. eine Probe aus einem biologischen Organismus, z.B. aus einer Körperflüssigkeit, eine Probe aus einer Zell- oder Mikroorganismenkultur etc. Weiterhin kann die Probe auch aus synthetischen Quellen, z.B. aus einer Synthesevorrichtung, stammen, oder ein Gemisch aus biologischem und synthetischem Material sein.

Die Probe kann vor dem Aufbringen auf den Träger gegebenenfalls prozessiert werden, z.B. durch enzymatische Reaktion, wie Amplifikation, Restriktionsspaltung, Markierung, Transkription, Translation, Fraktionierung, Vorreinigung etc. Die zu isolierenden Zielmoleküle können somit in markierter oder unmarkierter Form vorliegen.

Das erfindungsgemäße Verfahren wird an struktrierten Trägern, mit geschlossenen Kanälen durchgeführt. Die Kanäle sind beispielsweise Mikrokanäle mit einem Querschnitt von 10-10 000 µm. Beispiele für geeignete Träger mit Kanälen sind in WO00/13017 und WO00/13018 beschrieben. Vorzugsweise werden Träger verwendet, die zumindest teilweise im Bereich der Positionen mit den immobilisierten Rezeptoren optisch transparent oder/und elektrisch leitfähig sind.

Der für das erfindungsgemäße Verfahren eingesetzte mikrofluidische Träger enthält einen Array mit mehreren verschiedenen Rezeptoren, z.B. mit mindestens 10 und vorzugsweise mindestens 20 verschiedenen Rezeptoren. Die Rezeptoren werden vorzugsweise in situ schrittweise durch orts- oder/und zeitspezifisches Immobilisieren von Rezeptorbausteinen an den jeweils vorbestimmten Positionen auf dem oder im Träger aufgebaut.

An einer vorbestimmten Position kann nur eine einzige von Rezeptorspezies bzw. Rezeptorsequenz oder ein Gemisch von mehreren verschiedenen Rezeptorspezies bzw. Rezeptorsequenzen immobilisiert oder synthetisiert werden. Beispielsweise kann man Gemische von Rezeptorspezies verwenden, die für das gleiche zu extrahierende Zielmolekül spezifisch sind, z.B. ein Satz von verschiedenen Hybridisierungssonden für die Extraktion einer einzigen bestimmten Ziel-Nukleinsäure.

Das erfindungsgemäße Verfahren eignet sich insbesondere zum Einsatz als integriertes Synthese-Analyse(ISA)-Verfahren, d.h. ein Träger kann nach bestimmten Vorgaben in situ hergestellt und anschließend für einen Extraktionszyklus verwendet werden. Daran können sich gegebenenfalls weitere Synthese-Extraktions-Zyklen analog dem in WO00/13018 beschriebenen ISA-Prinzip anschließen. Auf diese Weise lassen sich mit einem Träger auch sehr unterschiedliche Moleküle, wie mRNA-Moleküle oder DNA-Sequenzen, extrahieren. Darüber hinaus ist eine Umstellung auf neue Extraktionsziele mit einem einzigen Träger möglich. Das Verfahren ist gut automatisierbar und kann mit weiteren nachfolgenden Prozessierungsschritten, wie einer Amplifikationsreaktion, z.B. unter Verwendung eines PCR-Thermocyclers, einer Detektion oder einer *in vitro* Translation kombiniert werden. Für diese nachfolgenden Prozessierungsschritte kann ein gemeinsamer, integrierter Reaktionsträger Anwendung finden.

Der Träger kann außer den eigentlichen Synthese/Reaktionsbereichen noch weitere mikrofluidische Funktionen bzw. Elemente beinhalten, wie etwa zusätzliche Reaktionsräume, z.B. für Enzymreaktionen innerhalb des Trägers, Reservoirs, Ventile, Pumpen, Thermoelemente, Reaktionsbereiche mit dort immobilisierten anderen biologisch-funktionalen Molekülen etc. Besonders bevorzugt sind Einrichtungen zur Temperierung des Trägers vorhanden, z.B. zum Einstellen einer bestimmten Temperatur oder eines bestimmten Temperaturprofils während des Extraktionsprozesses. Besonders bevorzugt enthält der Träger Thermoelemente, z.B. Peltier-Elemente, die eine orts- oder/und zeitspezifische Einstellung der Temperatur auf dem Träger oder einzelnen Bereichen des Trägers ermöglichen, für Hybridisierungen und nachfolgendes Aufschmelzen von Nukleinsäure-Doppelsträngen und für andere temperaturabhängige oder temperaturgesteuerte biochemische Reaktionen. Es können darüber hinaus auch Einrichtungen zur modulierten Zugabe von Puffern, z.B. aus unterschiedlichen Reservoirs, vorhanden sein, um Reaktionsbedingungen, z. B. Hybridisierungsbedingungen hinsichtlich der Stringenz, zeit- oder/und ortsspezifisch auf dem Träger oder Bereichen des Trägers zu modulieren.

Weiterhin kann der Träger zusätzliche funktionale biologische Moleküle in immobilisierter Form enthalten, beispielsweise Polymersonden, die zur Analyse der isolierten Zielmoleküle, z.B. zur Qualitätskontrolle, dienen. Weiterhin können funktionale biologische Moleküle in immobilisierter Form für enzymatische Reaktionen, z.B. Enzyme, PCR-Primer, Ribozyme etc., Modifikationen oder Derivatisierungen der Zielmoleküle vorliegen.

In einer weiteren Ausführungsform ist der Träger in einer Vorrichtung integriert, umfassend eine programmierbare Lichtquellenmatrix, eine Detektormatrix, einen vorzugsweise zwischen Lichtquellen- und Detektormatrix angeordneten Träger sowie Mittel zur Zufuhr von Fluids in den Träger und zur Ableitung von Fluids aus dem Träger. Die programmierbare Lichtquellen- bzw. Belichtungsmatrix kann eine Reflexionsmatrix, eine Lichtventilmatrix, z.B. eine LCD-Matrix oder eine selbstemittierende Belichtungsmatrix, sein. Derartige Lichtmatrices sind in WO00/13017 und WO00/13018 offenbart. Die Detektormatrix kann gegebenenfalls im Trägerkörper integriert sein.

Der in situ Aufbau von Rezeptoren auf dem Träger kann fluidchemische Schritte, photochemische Schritte, elektrochemische Schritte oder Kombinationen von zwei oder mehreren dieser Schritte umfassen. Ein Beispiel für eine elektrochemische Synthese von Rezeptoren auf einem Träger ist in DE 101 20 633.1 beschrieben. Ein Beispiel für ein Hybridverfahren, umfassend die Kombination von fluidchemischen Schritten und photochemischen Schritten, ist in DE 101 22 357.9 beschrieben.

Die Verwendung eines Trägers, umfassend einen Array mit einer Vielzahl verschiedener Rezeptoren, ermöglicht die Immobilisierung von mehreren unterschiedlichen Zielmolekülen aus einer komplexen Probe in einem einzigen Schritt. Die Elution der auf dem Träger immobilisierten Zielmoleküle kann.ebenfalls in einem einzigen Schritt erfolgen. Bevorzugt ist jedoch eine orts- oder/und zeitspezifische Elution, wobei einzelne Zielmoleküle oder einzelne Gruppen von Zielmolekülen zunächst in einem ersten Schritt von Träger - eluiert werden und die Elution weiterer Zielmoleküle oder Gruppen von Zielmolekülen in einem oder mehreren nachfolgenden Schritten erfolgt. Diese orts- oder/und zeitspezifische Elution umfasst vorzugsweise fluidchemische, photochemische oder elektrochemische Schritte oder Kombinationen davon. Ebenfalls bevorzugt ist die Verwendung von lokalen Temperaturänderungen auf dem Träger.

In einer besonders bevorzugten Ausführungsform der Erfindung erfolgt die Elution mittels einer ortsspezifischen Temperaturänderung, z.B. einer Temperaturerhöhung, die zu einer Denaturierung von Nukleinsäure-Doppelsträngen führt. Eine derartige ortsspezifische Temperaturänderung kann durch ortsspezifische Thermoelemente oder/und ortsaufgelöste Einwirkung von Strahlung, z.B. UV/VIS- und insbesondere IR-Strahlung, erfolgen, die ortsaufgelöst zu einer Wärmeentwicklung führt.

Eine separate orts- oder/und zeitspezifische Elution unterschiedlicher Zielmoleküle kann gegebenenfalls auch durch Abtrennung einzelner Reaktionsräume mittels hydrophober Barrieren entlang von Kanälen erreicht werden, um die diffusionsbedingten Vermischungen von eluierten Zielmolekülen, z.B. Nukleinsäuren, zu vermeiden.

Durch orts- oder/und zeitspezifische Elution können auch gezielt Gemische von ausgewählten Zielmolekülen erzeugt werden, wobei z.B. mehrere Fraktionen von Zielmolekülgemischen erhalten werden. Desweiteren können Zielmolekül-Gradienten bzw. Gradientenmischungen durch unterschiedliche Elutionseinwirkung an verschiedenen Stellplätzen, z.B. durch unterschiedlich lange Temperatureinwirkungen, hergestellt werden.

Das erfindungsgemäße Verfahren kann auch für die Isolierung von Proteinen und anderen Molekülen, insbesondere DNA-bindenden Molekülen, eingesetzt werden, wenn als Rezeptoren geeignete Fängersonden ausgewählt werden. In einer weiteren bevorzugten Ausführungsform können daher DNA-bindende Proteine mit Hilfe von DNA-Fängersonden, die als Doppel- oder Einzelstrang vorliegen können, extrahiert werden. In noch einer weiteren bevorzugten Ausführungsform können auch Peptidfängersonden für die Isolierung von Proteinen oder DNA-Molekülen verwendet werden. In noch einer weiteren bevorzugten Ausführungsform werden als Fängersonden Nukleinsäuren mit speziellen Bindungseigenschaften verwendet, z.B. Aptamere oder Ribozyme.

Das erfindungsgemäße Verfahren hat gegenüber dem Stand der Technik den Vorteil, dass es eine selektive Isolation von mehreren Zielmolekülen durch einen einzigen Arbeitsvorgang erlaubt. Die Verwendung mikrofluidischer Träger bewirkt, dass hierzu mit geringen Probenvolumina von z.B. 0,1 µl bis 100 µl gearbeitet werden kann. Trotz dieser geringen Probenvolumina werden im Träger hohe lokale Konzentrationen der Zielmoleküle erreicht, so dass sehr spezifisch und umfassend extrahiert werden kann.

Durch das mikrofluidische Format wird bei der Extraktion keine Aerosolbildung und dadurch Querkontamination hervorgerufen, wie es z.B. bei säulengestützten Verfahren mit Zentrifugationsschritt oder Vakuumabsaugung der Fall ist. Außerdem findet keine Kompetition von unterschiedlich häufigen oder/und in einer Tertiärstruktur vorliegenden Nukleinsäuren um einheitliche Bindungsstellen statt, welche ansonsten zum Vorteil der häufigeren bzw. weniger gefalteten Moleküle verlaufen kann, wie dies bei poly-A Säulen der Fall ist. Die durch das erfindungsgemäße Verfahren selektiv extrahierten Nukleinsäuren sind daher in anschließenden Schritten, wie z.B. PCR, leichter zu handhaben als ein Gemisch, wodurch weniger Probleme, z.B. falsche Bindung von Primern etc., hervorgerufen werden. Schließlich kann das erfindungsgemäße Verfahren auch leicht automatisiert werden. Insbesondere können vorher oder nachher stattfindende Prozesse automatisch angekoppelt werden, z.B. eine enzymatische Reaktion oder eine Ablage der isolierten Moleküle in geeigneten Behältern.

Ein zusätzlicher Vorteil der Verwendung mikrofluidischer Träger als Isolationsmatrix besteht darin, dass ein oder mehrere Zyklen der Bindung und anschließenden Elution von Zielmolekülen nach dem Durchflussprinzip durchgeführt werden können, wobei ein derartiger Zyklus die Schritte: Binden, Waschen unerwünschter Moleküle, Eluieren der gewünschten Moleküle und Rekonstituieren des Trägers umfasst. Die Isolation kann auch unter Zirkulation des Zielmolekülgemisches zur Verbesserung von aktiven Wechselwirkungen zwischen Rezeptor und Analyt bzw. Verbesserung des Waschvorgangs und der Rekonstitution erfolgen.

Die extrahierten Zielmoleküle können direkt oder indirekt für diagnostische oder therapeutische Zwecke eingesetzt werden. Weiterhin kann das extrahierte Material in Nachfolgereaktionen eingesetzt werden. So können aus extrahierten Nukleinsäuren Proteine oder Peptide, z.B. durch Transfer in geeignete Vektoren (Klonierung), oder in geeignete Zielzellen (Transformation bzw. Transfektion) oder durch *in vitro* Translation, insbesondere Isolierung von mRNA-Zielmolekülen, erzeugt werden.

Im Folgenden ist der schematische Ablauf des Verfahrens am Beispiel von Nukleinsäuren als Zielmolekülen erläutert:
1. Ermittlung von Daten zu den Sequenzen der Zielmoleküle, die aus einer Probe extrahiert werden sollen, z.B. 20 Gene aus einem Modellorganismus, der gerade untersucht wird; mit zumindest teilweise bekannten Sequenzen der 20 Gene;
2. Ermittlung von geeigneten Fängersonden, die komplementär zu singulären Bereichen in den ausgewählten Genen sind, bevorzugt mit Hilfe eines Computer-Algorithmus;
3. Vorbereitung der Probe durch Isolation von Gesamt-RNA aus Zellen des Modellorganismus;
4. Bereitstellen eines Geräts zur in situ Synthese von entsprechenden Fängersonden in oder auf einem mikrofluidischen Reaktionsträger;
5. Einspeisen der ermittelten Fängersonden-Sequenz in eine Synthese-Steuerungseinheit, die mit dem mikrofluidischen Träger integriert sein kann;
6. Synthetisieren der ausgewählten Fängersonden-Sequenz in oder auf dem mikrofluidischen Reaktionsträger;
7. Zugeben der Probe, so dass die Probe in die Kanäle des Reaktionsträgers gespült wird;
8. Equilibrieren der Bedingungen auf dem Träger, so dass eine Bindung zwischen Zielmolekülen und Fängersonden (Hybridisierung) bei geeigneten Pufferbedingungen und geeigneter Temperatur erfolgen kann;
9. Spülen der Kanäle, um ungebundenes Material zu entfernen;
10. Wechseln des Puffers (Stringenz) und eventuell der Temperatur, um die spezifischen Hybride zwischen Zielmolekülen und Fängersonden zu zerstören;
11. Gezieltes Ausspülen und Auffangen der abgelösten Zielmoleküle;
12. Weitere Verwendung der selektiv extrahierten Nukleinsäuren, z.B. in PCR und Klonierung, um dann nach Expression die Wirkung der Genprodukte zu studieren.

Das oben beschriebene Prinzip kann auch in abgewandelter Form zur selektiven Entfernung von bestimmten Komponenten (vorzugsweise mehr als eine Spezies) aus einer ein Zielmolekül oder ein Zielmolekül-Gemisch enthaltenden Probe verwendet werden. Störende Komponenten können entfernt werden, z.B. repetitive Elemente oder Telomersequenzen bei Analyse von Genfragmenten; Herausfiltern von bestimmten Genen (z.B. Housekeeping Genes) bei Transkriptionsanalysen; Proteine oder Proteinklassen, die die Proteomanalyse von (seltenen) Proteine stören. So können bekannte Komponenten abgefangen und nur noch erwünschte, z.B. bekannte oder unbekannte, Zielmoleküle eluiert werden. Das führt zu einer Aufkonzentration erwünschter Nukleinsäuren oder Proteine oder anderer erwünschter Biomoleküle.

Der Ablauf des Verfahrens bei einer derartigen Ausführungsform ist wie folgt:
1. Ermittlung von Daten zu den Sequenzen störender Komponenten;
2. Ermittlung geeigneter Fängersonden für störende Komponenten;
3.-7. wie oben;
8. Equilibrieren bei Bedingungen, bei denen störende Komponenten binden;
9. Gezieltes Ausspülen und Auffangen der gewünschten Moleküle (störende Elemente verbleiben auf dem Träger).

Durch diese Vorgehensweise werden störende Komponenten abgereichert, so dass nachfolgende Analyse der gewünschten Moleküle mit höherer Präzision durchgeführt werden können. Weiterhin wird eine Aufkonzentrierung der gewünschten Zielmoleküle der zu untersuchenden Probe erreicht und störende Moleküle, z.B. mehrere Spezies parallel, können gezielt aus dem Molekülgemisch entfernt werden.

Ein weiterer Gegenstand der Erfindung ist somit ein Verfahren zur Isolierung von Zielmolekülen aus einer Probe gemäß Anspruch 19.

Die Verfahrensführung bei dieser Ausführungsform erfolgt entsprechend den Erläuterungen für die erste Ausführungsform, mit der Maßgabe, dass die in der Probe vorhandenen Zielmoleküle nicht oder in geringerem Umfang als die störenden Komponenten an die Rezeptoren auf dem Träger binden.

Die vorliegende Erfindung umfasst schließlich auch eine Ausführungsform betreffend ein Verfahren zur Isolierung von Zielmolekülen aus einer Probe, wobei mindestens ein Verfahrenszyklus, bei dem die Zielmoleküle an die Rezeptoren binden und mindestens einen Verfahrenszyklus, bei dem die Zielmoleküle nicht oder nur gering an die Rezeptoren binden, kombiniert werden. Vorzugsweise wird zunächst ein Verfahrenszyklus durchgeführt, bei dem die Zielmoleküle nicht oder nur gering binden, an den sich ein Verfahrenszyklus anschließt, bei dem die Zielmoleküle binden und dann ort- oder/und zeitspezifisch vom Träger eluiert werden.

Weiterhin soll die Erfindung durch die folgenden Abbildungen näher erläutert werden.

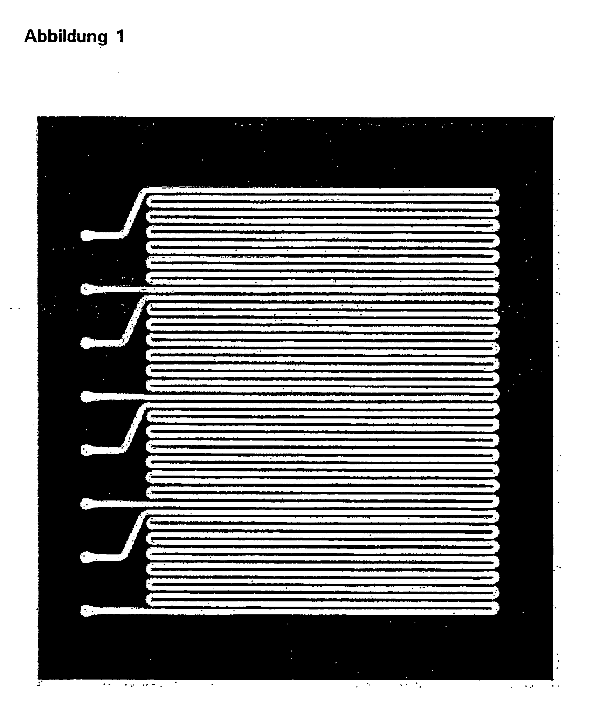

**Abbildung 1** zeigt ein Beispiel für einen geeigneten mikrofluidischen Reaktionsträger mit insgesamt 4 separaten Kanälen.

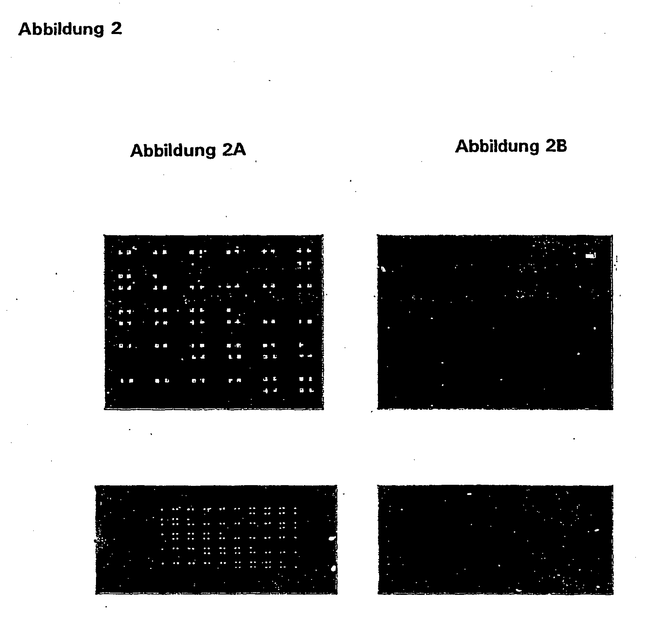

**Abbildung 2** zeigt die spezifische Hybridisierung von Nukleinsäure-Zielmolekülen an einem mikrofluidischen Reaktionsträger und deren quantitative Extrahierbarkeit. In **Abbildung 2A** ist die Hybridisierung eines Zielmoleküls an vorbestimmten Posititonen auf einem mikrofluidischen Reaktionsträger erkennbar. **Abbildung 2B** zeigt den Träger nach Extraktion. Es ist zu erkennen, dass eine quantitative Elution des Zielmoleküls erfolgt ist.

## Patentansprüche

1. Verfahren zur Isolierung von Zielmolekülen aus einer Probe, umfassend die Schritte:
(a) Bereitstellen eines mikrofluidischen Trägers mit geschlossenen Kanälen und einem Array von mehreren verschiedenen Rezeptoren, die auf jeweils unterschiedlichen Positionen auf dem oder im Träger immobilisiert sind,
(b) Leiten einer Probe, die mehrere unterschiedlich zu isolierende Zielmoleküle enthält, durch den mikrofluidischen Träger, unter Bedingungen, bei denen Zielmoleküle spezifisch an die auf dem Träger immobilisierten Rezeptoren binden können,
(c) Entfernen von nichtgebundenem Material aus der Probe vom Träger und
(d) Ortsspezifischen Eluieren der mehreren unterschiedlichen an den Träger gebundenen Zielmoleküle in einem einzigen Schritt, wodurch mehrere Spezies von Zielmolekülen parallel isoliert werden und ein Gemisch von ausgewählten Zielmolekülen erzeugt wird.

2. Verfahren nach Anspruch 1,
**dadurch gekennzeichnet,**
**dass** die Zielmoleküle ausgewählt werden aus Nukleinsäuren, Polypeptiden, Peptiden und Sacchariden.

3. Verfahren nach Anspruch 1 oder 2,
**dadurch gekennzeichnet,**
**dass** die Zielmoleküle aus Nukleinsäuren, insbesondere DNA- oder/und RNA-Molekülen ausgewählt werden.

4. Verfahren nach Anspruch 3,
**dadurch gekennzeichnet,**
**dass** als Rezeptoren Hybridisierungssonden verwendet werden.

5. Verfahren nach Anspruch 4,
**dadurch gekennzeichnet,**
**dass** man als Hybridisierungssonden Nukleinsäuren oder Nukleinsäureanaloga verwendet.

6. Verfahren nach Anspruch 4 oder 5,
**dadurch gekennzeichnet,**
**dass** die Hybridisierungssonden eine Länge entsprechend 10-100 Nukleotiden aufweisen.

7. Verfahren nach einem der vorhergehenden Ansprüche,
**dadurch gekennzeichnet,**
**dass** man eine Probe biologischen oder/und synthetischen Ursprungs verwendet.

8. Verfahren nach einem der vorhergehenden Ansprüche,
**dadurch gekennzeichnet,**
**dass** man eine Probe verwendet, die einem oder mehreren Vorbehandlungsschritten unterzogen worden ist.

9. Verfahren nach Anspruch 1,
**dadurch gekennzeichnet,**
**dass** der Träger Mikrokanäle mit einem Durchmesser von 10-1000 µm aufweist.

10. Verfahren nach einem der vorhergehenden Ansprüche,
**dadurch gekennzeichnet,**
**dass** der Array auf dem Träger mindestens 10, vorzugsweise mindestens 20 Positionen mit verschiedenen Rezeptoren enthält.

11. Verfahren nach einem der vorhergehenden Ansprüche,
**dadurch gekennzeichnet,**
**dass** die Rezeptoren an den einzelnen Positionen Einzelsequenzen oder/und Sequenzgemische enthalten.

12. Verfahren nach einem der vorhergehenden Ansprüche,
**dadurch gekennzeichnet,**
**dass** die Rezeptoren des Arrays in situ schrittweise durch orts- oder/und zeitspezifisches Immobilisieren von Rezeptorbausteinen an den jeweils vorbestimmten Positionen auf dem oder im Träger aufgebaut werden.

13. Verfahren nach Anspruch 12,
**dadurch gekennzeichnet,**
**dass** man den Träger für einen oder mehrere integrierte Synthese-Analyse-Zyklen einsetzt.

14. Verfahren nach Anspruch 12 oder 13,
**dadurch gekennzeichnet,**
**dass** man den Träger zusammen mit einer programmierbaren Lichtquellenmatrix und einer Detektionsmatrix verwendet.

15. Verfahren nach einem der vorhergehenden Ansprüche,
**dadurch gekennzeichnet,**
**dass** man die extrahierten Zielmoleküle einer Nachfolgereaktion unterzieht.

16. Verfahren nach einem der vorhergehenden Ansprüche,
**dadurch gekennzeichnet,**
**dass** die extrahierten Zielmoleküle zur direkten oder indirekten diagnostischen oder therapeutischen Verwendung vorgesehen sind.

17. Verfahren nach einem der vorhergehenden Ansprüche,
**dadurch gekennzeichnet,**
**dass** ein Träger verwendet wird, der zusätzliche funktionale biologische Moleküle in immobilisierter Form enthält.

18. Verfahren nach Anspruch 17,
**dadurch gekennzeichnet,**
**dass** die zusätzlichen funktionalen biologischen Moleküle Polymersonden sind und der Analyse der isolierten Zielmoleküle dienen.

19. Verfahren zur Isolierung von Zielmolekülen aus einer Probe, umfassend mindestens einen Verfahrenszyklus nach Anspruch 1 und mindestens einen Verfahrenszyklus umfassend die Schritte:
(a) Bereitstellen eines mikrofluidischen Trägers mit geschlossenen Kanälen und einem Array von mehreren verschiedenen Rezeptoren, die auf jeweils unterschiedlichen Positionen auf dem oder im Träger immobilisiert sind,
(b) Leiten einer Probe, die zu isolierende Zielmoleküle enthält, durch den mikrofluidischen Träger, unter Bedingungen, bei denen störende Komponenten aus einer Probe spezifisch an die auf dem Träger immobilisierten Rezeptoren binden können, und wobei die in der Probe vorhandenen Zielmoleküle nicht oder in geringerem Umfang als die störenden Komponenten an die Rezeptoren auf dem Träger binden, und wobei mehr als eine Spezies störender Komponenten an die auf dem Träger immobilisierten Rezeptoren bindet, und
(c) Ausspülen und Auffangen der nichtgebundenen Zielmoleküle vom Träger.

## Claims

1. Method for isolating target molecules from a sample comprising the steps:
(a) providing a microfluidic carrier with closed channels and an array of a plurality of different receptors which are immobilized in each case at different positions on or in the carrier,
(b) leading a sample which contains a plurality of different target molecules to be isolated, through the microfluidic carrier under conditions in which target molecules can specifically bind to the receptors immobilized on the carrier,
(c) removing unbound material of the sample from the carrier and
(d) site-specifically eluting the plurality of different target molecules bound to the carrier in a single step by means of which a plurality of species of target molecules are isolated in parallel and a mixture of selected target molecules is obtained.

2. Method according to claim 1,
**characterized in that**
the target molecules are selected from nucleic acids, polypeptides, peptides and saccharides.

3. Method according to claim 1 or 2,
**characterized in that**
the target molecules are selected from nucleic acids, in particular DNA molecules or/and RNA molecules.

4. Method according to claim 3,
**characterized in that**
hybridization probes are used as receptors.

5. Method according to claim 4,
**characterized in that**
nucleic acids or nucleic acid analogues are used as hybridization probes.

6. Method according to claim 4 or 5,
**characterized in that**
the hybridization probes have a length corresponding to 10 - 100 nucleotides.

7. Method according to one of the previous claims,
**characterized in that**
a sample of biological or/and synthetic origin is used.

8. Method according to one of the previous claims,
**characterized in that**
a sample is used which has been subjected to one or more pretreatment steps.

9. Method according to claim 1,
**characterized in that**
the carrier has microchannels of 10 - 1000 µm in diameter.

10. Method according to one of the previous claims,
**characterized in that**
the array on the carrier contains at least 10 and preferably at least 20 positions with different receptors.

11. Method according to one of the previous claims,
**characterized in that**
the receptors at the individual positions contain single sequences or/and sequence mixtures.

12. Method according to one of the previous claims,
**characterized in that**
the receptors of the array are constructed stepwise in situ on or in the carrier by site-specific or/and time-specific immobilization of receptor building blocks at the respective predetermined positions.

13. Method according to claim 12,
**characterized in that**
the carrier is used for one or more integrated synthesis-analysis cycles.

14. Method according to claim 12 or 13,
**characterized in that**
the carrier is used together with a programmable light source matrix and a detection matrix.

15. Method according to one of the previous claims,
**characterized in that**
the extracted target molecules are subjected to a subsequent reaction.

16. Method according to one of the previous claims,
**characterized in that**
the extracted target molecules are intended for a direct or indirect diagnostic or therapeutic use.

17. Method according to one of the previous claims,
**characterized in that**
a carrier is used which contains additional functional biological molecules in an immobilized form.

18. Method according to claim 17,
**characterized in that**
the additional functional biological molecules are polymer probes and are used to analyse the isolated target molecules.

19. Method for isolating target molecules from a sample comprising at least one process cycle according to claim 1 and at least one process cycle comprising the steps:
(a) providing a microfluidic carrier with closed channels and an array of a plurality of different receptors which are immobilized in each case at different positions on or in the carrier,
(b) leading a sample which contains target molecules to be isolated through the microfluidic carrier under conditions in which interfering components from a sample can specifically bind to the receptors immobilized on the carrier, and the target molecules present in the sample do not bind to the receptors on the carrier or to a lesser extent than the interfering components, and more than one species of interfering components binds to the receptors immobilized on the carrier, and
(c) eluting and collecting unbound target molecules from the carrier.

## Revendications

1. Procédé d'isolement de molécules cibles à partir d'un échantillon, comprenant les étapes consistant à :
(a) se munir d'un support microfluidique comportant des canaux fermés et une série de plusieurs récepteurs différents qui sont immobilisés chacun en une position différente sur le support ou dans celui-ci,
(b) faire passer un échantillon qui contient plusieurs molécules cibles à isoler de façon différente à travers le support microfluidique dans des conditions dans lesquelles les molécules cibles peuvent se lier spécifiquement aux récepteurs immobilisés sur le support,
(c) éliminer du support du matériau non lié de l'échantillon et
(d) procéder à une élution spécifique à un site des différentes molécules cibles liées au support, dans une étape unique de sorte que plusieurs espèces de molécules cibles sont isolées en parallèle et un mélange de molécules cibles choisies est produit.

2. Procédé selon la revendication 1, **caractérisé en ce que** les molécules cibles sont choisies parmi les acides nucléiques, les polypeptides, les peptides et les saccharides.

3. Procédé selon la revendication 1 ou 2, **caractérisé en ce que** les molécules cibles sont choisies parmi les acides nucléiques, en particulier, les molécules d'ADN et/ou d'ARN.

4. Procédé selon la revendication 3, **caractérisé en ce que** l'on utilise comme récepteurs, des sondes d'hybridation.

5. Procédé selon la revendication 4, **caractérisé en ce que** l'on utilise, comme sondes d'hybridation, des acides nucléiques ou des analogues d'acide nucléique.

6. Procédé selon la revendication 4 ou 5, **caractérisé en ce que** les sondes d'hybridation présentent une longueur de 10 à 100 nucléotides.

7. Procédé selon l'une des revendications précédentes, **caractérisé en ce que** l'on utilise un échantillon d'origine biologique et/ou synthétique.

8. Procédé selon l'une des revendications précédentes, **caractérisé en ce que** l'on utilise un échantillon qui est soumis à une ou plusieurs étapes de prétraitement.

9. Procédé selon la revendication 1, **caractérisé en ce que** les microcanaux du support présentent un diamètre de 10 à 1 000 µm.

10. Procédé selon l'une des revendications précédentes, **caractérisé en ce que** la série sur le support contient au moins 10, de préférence au moins 20 positions avec des récepteurs différents.

11. Procédé selon l'une des revendications précédentes, **caractérisé en ce que** les récepteurs contiennent au niveau des positions individuelles, des séquences individuelles et/ou des mélanges de séquences.

12. Procédé selon l'une des revendications précédentes, **caractérisé en ce que** les récepteurs de la série sont montés *in situ* par étape sur ou dans le support par immobilisation spécifique au site et/ou au temps, d'éléments de récepteur sur chacune des positions prédéterminées.

13. Procédé selon la revendication 12, **caractérisé en ce que** l'on utilise le support pour un ou plusieurs cycles de synthèse - analyse intégrés.

14. Procédé selon la revendication 12 ou 13, **caractérisé en ce que** l'on utilise le support conjointement avec une matrice de source lumineuse programmable et une matrice de détection.

15. Procédé selon l'une des revendications précédentes, **caractérisé en ce qu'**on soumet la molécule cible extraite à une réaction subséquente.

16. Procédé selon l'une des revendications précédentes, **caractérisé en ce que** les molécules cibles extraites sont prévues pour l'utilisation diagnostique ou thérapeutique directe ou indirecte.

17. Procédé selon l'une des revendications précédentes, **caractérisé en ce que** l'on utilise un support qui contient des molécules biologiques fonctionnelles supplémentaires sous forme immobilisée.

18. Procédé selon la revendication 17, **caractérisé en ce que** les molécules biologiques fonctionnelles supplémentaires sont des sondes polymères et servent à l'analyse des molécules cibles isolées.

19. Procédé d'isolement de molécules cibles d'un échantillon, comprenant au moins un cycle du procédé selon la revendication 1 et au moins un cycle du procédé comprenant les étapes consistant à :
(a) se munir d'un support microfluidique comportant des canaux fermés et une série de plusieurs récepteurs différents qui sont immobilisés chacun en une position différente sur le support ou dans celui-ci,
(b) faire passer un échantillon qui contient des molécules cibles à isoler à travers le support microfluidique dans des conditions dans lesquelles les composants gênants d'un échantillon peuvent se lier spécifiquement aux récepteurs immobilisés sur le support et dans lequel les molécules cibles présentes dans l'échantillon ne se lient pas ou se lie dans une moindre mesure que les composants gênants, aux récepteurs sur le support, et dans lequel plusieurs espèces de composants gênants se lient aux récepteurs immobilisés sur le support, et
(c) rincer et recueillir les molécules cibles non liées du support.
